(19) Europäisches Patentamt
European Patent Office
Office européen des brevets

(11) EP 0 579 615 B1

(12) **EUROPÄISCHE PATENTSCHRIFT**

(45) Veröffentlichungstag und Bekanntmachung des Hinweises auf die Patenterteilung:
**04.07.2001 Patentblatt 2001/27**

(21) Anmeldenummer: **92905643.0**

(22) Anmeldetag: **05.03.1992**

(51) Int Cl.7: **C07D 451/10**, C07D 451/14, A61K 31/46

(86) Internationale Anmeldenummer:
**PCT/EP92/00489**

(87) Internationale Veröffentlichungsnummer:
**WO 92/16528 (01.10.1992 Gazette 1992/25)**

(54) **NEUE ESTER BI- UND TRICYCLISCHER AMINOALKOHOLE, IHRE HERSTELLUNG UND IHRE VERWENDUNG IN ARZNEIMITTELN**

NEW BI- AND TRICYCLIC AMINOALCOHOL ESTERS, THEIR PREPARATION AND THEIR USE IN MEDICAMENTS

ESTERS NOUVEAUX D'AMINO-ALCOOLS BICYCLIQUES ET TRICYCLIQUES, LEUR PREPARATION ET LEUR UTILISATION DANS DES MEDICAMENTS

(84) Benannte Vertragsstaaten:
**AT BE CH DE DK ES FR GB GR IT LI LU MC NL SE**

(30) Priorität: **15.03.1991 DE 4108393**

(43) Veröffentlichungstag der Anmeldung:
**26.01.1994 Patentblatt 1994/04**

(73) Patentinhaber:
- **Boehringer Ingelheim Pharma KG**
  **55218 Ingelheim am Rhein (DE)**
  Benannte Vertragsstaaten:
  **BE CH DE DK ES FR GR IT LI LU MC NL SE AT**
- **Boehringer Ingelheim International GmbH**
  **55218 Ingelheim am Rhein (DE)**
  Benannte Vertragsstaaten:
  **GB**

(72) Erfinder:
- **BANHOLZER, Rolf**
  **D-6507 Ingelheim am Rhein (DE)**
- **BAUER, Rudolf**
  **D-6531 Ockenheim (DE)**
- **REICHL, Richard**
  **D-6535 Gau-Algesheim (DE)**

(74) Vertreter: **Laudien, Dieter, Dr.**
**Boehringer Ingelheim GmbH,**
**Binger Strasse 173**
**55216 Ingelheim (DE)**

(56) Entgegenhaltungen:

| | |
|---|---|
| **EP-A- 418 716** | **EP-A- 0 128 437** |
| **DE-A- 1 620 077** | **DE-A- 1 770 183** |
| **DE-A- 2 540 633** | **FR-A- 3 228** |
| **FR-A- 2 168 881** | **FR-A- 2 208 649** |
| **US-A- 2 753 288** | **US-A- 3 145 211** |

- **Acta Chemica Scandinavica, Band 24, Nr. 5, 1970, (Copenhagen, DK), K. NYBERG et al.: "Investigations of dithienylglycolic esters. I. Preparation of methylyl dithienylglycolates. Magnetically nonequivalent protons in dithienylglycolates", Seiten 1590-1596, siehe Seite 1595**
- **Arzneimittelforschung, Band 17, Nr. 6, 1967, (Aulendorf, DE), H. BERTHOLDT et al.: "Über Azoniaspiro-Verbindungen", Seiten 719-726, siehe Seite 722**
- **Journal of the Chemical Society, 1957, (Cambridge, US), R. FOSTER et al.: "Further new tropine derivatives", Seiten 3575-3578, siehe Seite 3576**
- **S. BUDAVARI ET AL.: "The Merck Index", 1996, MERCK RESEARCH LABORATORIES, WHITEHOUSE STATION N.J.**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 579 615 B1

Printed by Jouve, 75001 PARIS (FR)

## Beschreibung

Die Erfindung betrifft neue Ester von bi- und tricyclischen Aminoalkoholen, die Herstellung dieser Verbindungen und ihre Verwendung in Arzneimitteln.

Die neuen Verbindungen entsprechen der Formel

$$A - O - CO - Z \qquad (I)$$

in der

A eine Gruppe der Formel

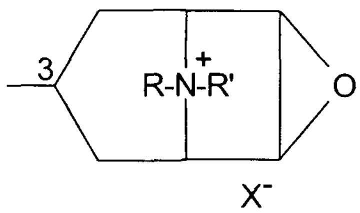

oder

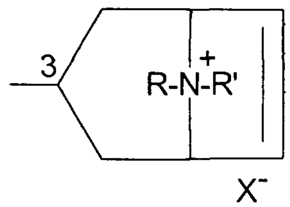

in der 3-α-Form steht, worin
einer der Reste R und R' Methyl, der andere Methyl, Ethyl, n-Propyl oder i-Propyl bedeuten

$X^-$ für ein Äquivalent eines Anions, und

Z für die Gruppe

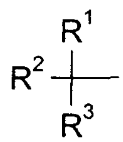

(III)

steht, worin

$R_1$ OH, Methyl oder $CH_2OH$,
$R_2$ und $R_3$, die gleich oder verschieden sein können und von denen eines auch H sein kann,

a) Phenyl-, Furyl, Thienyl, $C_5$-$C_7$-Cycloalkyl, Pyridyl, $C_5$-$C_7$-Cycloalkenyl, oder

b) einen gegebenenfalls sauerstoffunterbrochenen aliphatischen Rest mit bis zu 20 C-Atomen, einen phenyl-, phenoxy-, thienyl-, furyl-, $C_5$-$C_7$-cycloalkyl- oder fluorsubstituierten $C_1$-$C_6$-Alkylrest bedeutet,

oder die gesamte Gruppe III auch für einen tricyclischen Rest der Formel

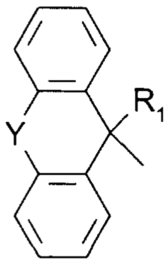

oder

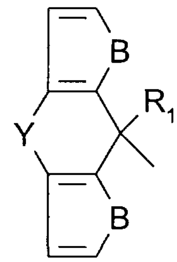

(IV) (V)

oder einen Rest der Formel

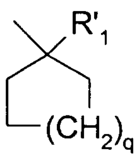

(VI)

steht, wobei B S oder CH=CH, $R'_1$ das gleiche wie $R_1$ und zusätzlich Phenyl, Thienyl, Furyl, Thiazolyl, Thiadiazolyl oder Methoxyphenyl,
Y eine Einfachbindung, ein O- oder S-Atom oder eine der Gruppen $-CH_2-$, $-CH_2-CH_2-$, -CH=CH-, $-OCH_2-$ oder $-SCH_2-$ und q 1, 2 oder 3 repräsentiert,

mit der Maßgabe,

a) daß Z-CO nicht für den Tropasäurerest steht, wenn A

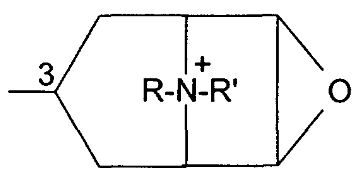

und R und R' Methyl oder R Methyl und R' Ethyl bedeuten;

b) daß wenn $R_1$ OH oder Methyl und einer der Reste $R_2$ oder $R_3$ Thienyl bedeuten, der andere der Reste $R_2$ oder $R_3$ nicht gleichzeitig Thienyl, Phenyl, Furyl, Cyclopentyl oder Cyclohexyl sein kann;

Dementsprechend steht die Gruppe A z.B. für die Reste des Scopins, des N-Ethylnorscopins, des N-Isopropylnorscopins, des 6,7-Dehydrotropins, des N-Isopropyl-6,7-Dehydronortropins bzw. die entsprechenden Quartärverbindungen, wobei das Anion bevorzugt Br - bzw. $CH_3SO_3^{\ominus}$ ist.
Die Gruppe Z kann beispielsweise folgende Bedeutungen haben, wobei die aromatischen Reste auch substituiert sein können, z.B. durch $CH_3$, $OCH_3$, F, Cl:

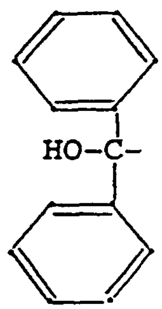

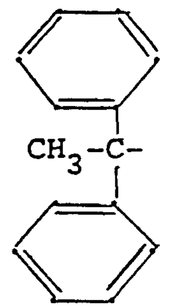

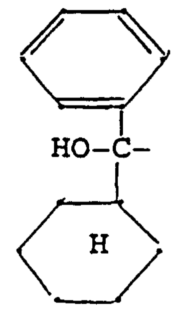

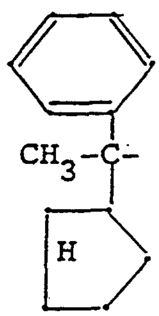

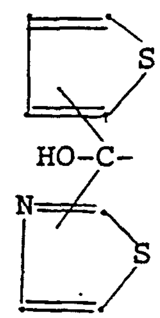

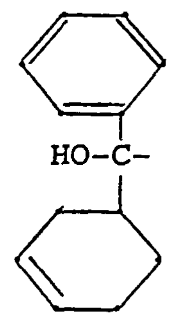

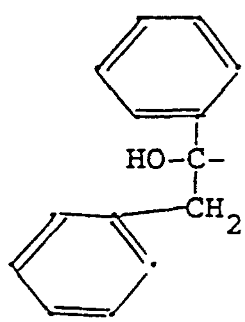

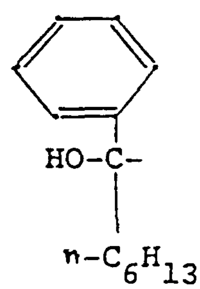

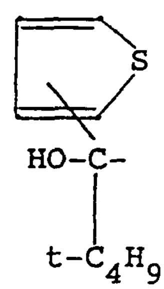

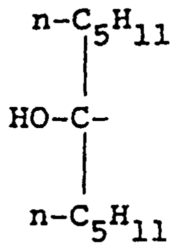
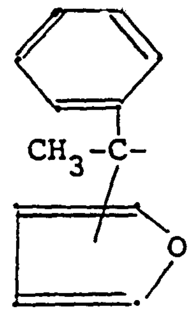

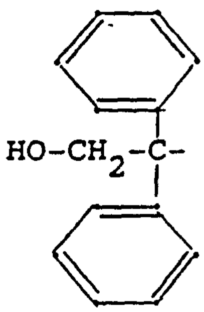
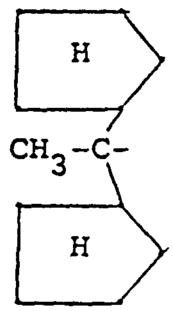
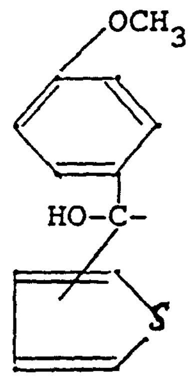

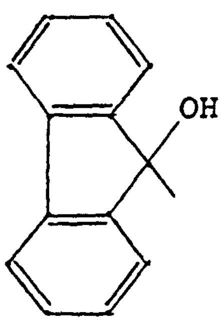
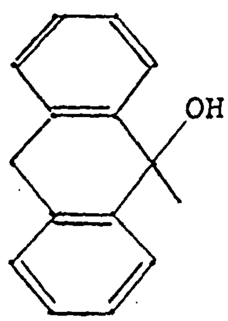
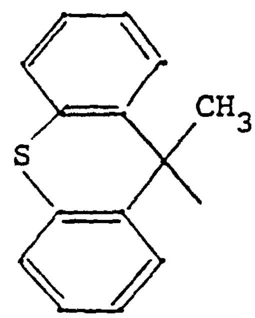

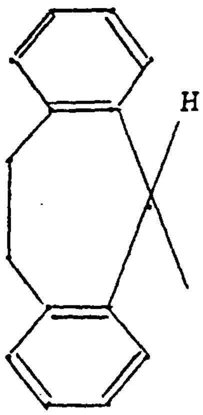
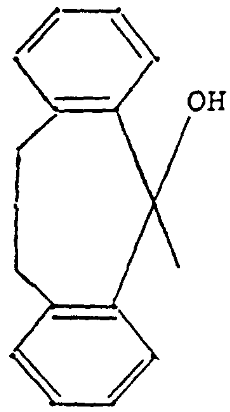
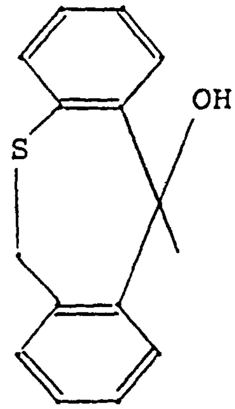

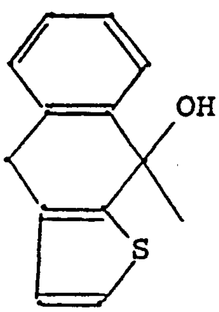
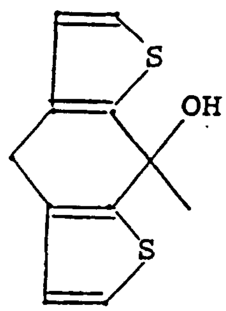
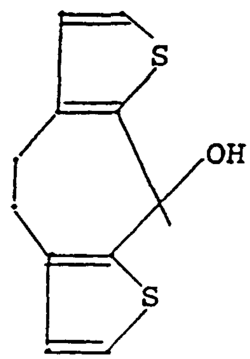

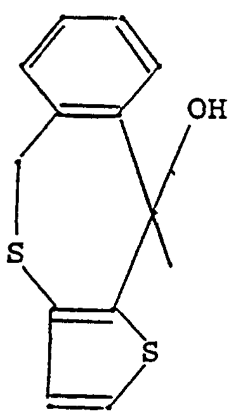
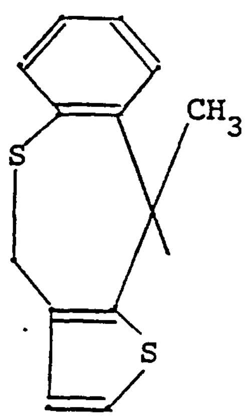
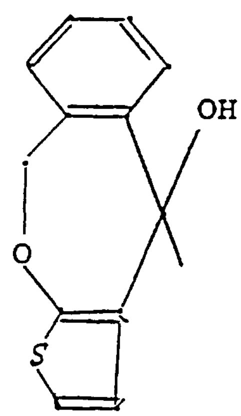

Für die therapeutische Anwendung eignen sich besonders die Quartärverbindungen der Formel I, während die Tertiärverbindungen außer als Wirkstoffe auch als Zwischenprodukte wichtig sind.
Die erfindungsgemäßen Verbindungen stellen stark und lange wirksame Antich olinergika dar. Bei Dosierungen im µg-Bereich werden inhalativ Wirkungsdauern von mehr als 24 Stunden erreicht. Die Toxizität liegt zudem im gleichen Bereich wie bei dem Handelsprodukt Ipratropiumbromid, während gleichzeitig die therapeutische Wirkung z. T. deutlich stärker ist.

Die neuen Verbindungen eignen sich, entsprechend ihrer Natur als Anticholinergika, z.B zur Behandlung von chronisch obstruktiver Bronchitis und (leichtem bis mittelschwerem) Asthma, ferner zur Behandlung vagal bedingter Sinusbradykardien.
Während sich bei Atemwegserkrankungen hauptsächlich die inhalative Anwendung der neuen Wirkstoffe (insbesondere der Quartärverbindungen) empfiehlt, wodurch Nebenwirkungen weitgehend ausgeschaltet werden, erfolgt die Anwendung bei Sinusbradykardien vorzugsweise intravenös oder oral. Dabei erweist sich als vorteilhaft, daß die neuen Verbindungen die Magen/Darm-Motilität weitgehend unbeeinflußt lassen.

Für die Applikation werden die erfindungsgemäßen Verbindungen mit bekannten Hilfs- und/oder Trägerstoffen zu gebräuchlichen galenischen Zubereitungen verarbeitet, z.B. zu Inhalationslösungen, Suspensionen in verflüssigten Treibgasen, Liposomen bzw. Proliposomen enthaltenden Zubereitungen, Injektionslösungen, Tabletten, Dragées, Kapseln, Inhalationspulvern zur Anwendung in üblichen Inhalationsgeräten.

Formulierungsbeispiele (Angaben in Gewichtsprozent):

| 1. Dosieraerosol | |
|---|---|
| Wirkstoff gemäß der Erfindung | 0,005 |
| Sorbitantrioleat | 0,1 |
| Monofluortrichlormethan und Difluordichlormethan 2 : 3 | ad 100 |

Die Suspension wird in einen üblichen Aerosolbehälter mit Dosierventil gefüllt. Pro Betätigung werden vorzugsweise 50 µl Suspension abgegeben. Der Wirkstoff kann gewünschtenfalls auch höher dosiert werden (z.B. 0.02 Gew.-%).

| 2. Tabletten | |
|---|---|
| Wirkstoff gemäß der Erfindung | 0,05 |
| Kolloidale Kieselsäure | 0,95 |
| Milchzucker | 65,00 |
| Kartoffelstärke | 28,00 |
| Polyvinylpyrrolidon | 3,00 |
| Na-Celluloseglykolat | 2,00 |
| Magnesiumstearat | 1,00 |

Die Bestandteile werden in üblicher Weise zu Tabletten von 200 mg verarbeitet.

Die vorteilhaften Eigenschaften der neuen Verbindungen zeigen sich beispielsweise in der Hemmung der Broncholyse am Kaninchen (Acetylcholinspasmus i.v.). Nach intravenöser Gabe der neuen Wirkstoffe (Dosis 3 µg/kg i.v.) trat die maximale Wirkung nach 10 bis 40 Minuten ein. Auch am isolierten Organ, z.B. am Meerschweinchenileum oder -rektum konnte eine lange Wirkungsdauer für zahlreiche erfindungsgemäße Verbindungen gezeigt werden.

Zur Herstellung der neuen Verbindungen dienen an sich bekannte Verfahren.

1. Bevorzugt wird ein Ester der Formel

$$Z - CO - OR'' \qquad (VII),$$

worin Z die obige Bedeutung hat und R" für einen $C_1$-$C_4$-Alkylrest, vorzugsweise für Methyl oder Ethyl steht, mit einem Aminoalkohol der Formel

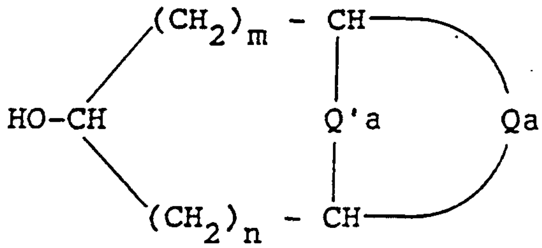

(VIII),

worin m und n die obige Bedeutung haben, $Q'_a$ NR' oder NH bedeutet, $Q_a$ für eine der zweibindigen Gruppen -CH=CH- oder

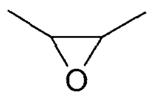

steht und worin die OH-Gruppe sich in α- oder β-Stellung befindet, in Gegenwart eines üblichen Umesterungskatalysators umgeestert oder

2.) ein reaktionsfähiges Derivat (R''' leicht abspaltbare Gruppe)

$$Z - CO - OR''' \quad (IX)$$

der Säure Z-CO-OH, insbesondere ein Säurechlorid oder Imidazolid, mit einem Alkohol der Formel VIII, gegebenenfalls im Überschuß oder in Gegenwart eines tertiären Amins wie Triethylamin umgesetzt,
und die erhaltene Verbindung gegebenenfalls

a) wenn $Q'_a$ NR' bedeutet, mit einem reaktionsfähigen Monoderivat X-R eines entsprechenden Alkans (X = Abgangsgruppe) quaterniert oder
b) wenn $Q'_a$ NH bedeutet, mit einem endständig disubstituierten Alkan X-($C_4$-$C_6$-Alkylen)-X ohne Zwischenisolierung quaterniert

Alternativ können Ausgangsverbindungen quaterniert werden, in denen die Ausgangsverbindung VIII am Stickstoff statt R' bereits R in der Bedeutung "halogen- oder hydroxysubstituierter Alkylrest" enthalten.
Die Umesterung nach Verfahren 1 wird in der Wärme in einem organischen Lösungsmittel, z.B. Toluol, Xylol, Heptan, oder in der Schmelze durchgeführt, wobei starke Basen wie Natriummethylat, Natriumethylat, Natriumhydrid, metallisches Natrium, als Katalysator dienen. Zur Entfernung des freigesetzten niederen Alkohols aus dem Gleichgewicht wird verminderter Druck angewendet, ggf. der Alkohol azeotrop abdestilliert. Die Umesterung erfolgt bei Temperaturen, die im allgemeinen 95°C nicht überschreiten. Häufig verläuft die Umesterung in der Schmelze günstiger. Die Umsetzung gemäß Verfahren 2 erfolgt in einem organischen Lösungsmittel bzw. Lösungsmittelgemisch, das unter den Reaktionsbedingungen hinreichend inert ist, z.B. Aceton, Acetonitril, bei Temperaturen zwischen etwa 0°C und der Siedetemperatur des Reaktionsgemischs.
Aus Säureadditionssalzen der tertiären Amine kann man gewünschtenfalls mit geeigneten basischen Verbindungen in an sich bekannter Weise die freien Basen erhalten. Die Quaternierung wird in geeigneten Lösungsmitteln, etwa Acetonitril oder Acetonitril/Methylenchlorid vorzugsweise bei Raumtemperatur durchgeführt; dabei wird als Quaternierungsreagenz bevorzugt ein entsprechendes Alkylhalogenid, z.B. Alkylbromid, oder auch ein entsprechendes Sulfonsäurederivat, etwa ein Methan- oder Toluolsulfonsäurederivat, verwendet.
Die Überführung in die tertiäre und dann quartäre Verbindung erfolgt dann mit Hilfe geeigneter 1,4-, 1,5- bzw. 1,6-Dihalogenalkane ohne Zwischenisolierung.
Die Ausgangsstoffe können - soweit sie nicht schon beschrieben wurden - analog zu bekannten Verbindungen erhalten werden.

Beispiele:

Di-(2-thienyl)glykolsäuremethylester aus Oxalsäuredimethylester und 2-Thienylmagnesiumbromid; Di-(2-thienyl)glykolsäureethylester aus (2-Thienyl)glyoxylsäure und 2-Thienyllithium; Hydroxy-phenyl-(2-thienyl)essigsäureethylester aus Phenylglyoxylsäuremethylester und 2-Thienylmagnesiumbromid oder aus (2-Thienyl)glyoxylsäuremethylester und Phenylmagnesiumbromid.
Ähnlich können 2-Thienylglyoxylsäuremethylester und Cyclohexyl- bzw. Cylopentylmagnesiumbromid umgesetzt werden.
Auch für die Herstellung der Aminoalkohole stehen mehrere Verfahren zur Verfügung.
Pseudoscopin kann nach M. Polonovski et al., Bull. soc. chim. 43, 79 (1928) erhalten werden.
Pseudotropenol kann aus dem Gemisch isoliert werden (fraktionierte Kristallisation bzw. Destillation), das z. B. nach V. Hayakawa et al., J. Amer.Chem.Soc. 1978, 100(6), 1786 bzw. R. Noyori et al., J.Amer.Chem.Soc. 1974, 96 (10), 3336 erhalten wird.
N-Ethylnorscopin und N-Isopropylnorscopin können durch Hydrogenolyse aus den entsprechenden N-Alkylnorscopolaminen analog Banholzer DE-A P 3215933 hergestellt werden. 6-Methyl-6-azabicyclo[3.2.1]octan-3-$\alpha$-ol läßt sich herstellen nach F.I.Carroff et al., J. Med.Chem. 30, 805 (1987),
7-Methyl-7-azabicyclo[2.2.1]heptan-2$\alpha$-ol nach J.R. Pfister et al., J. Pharmac. Sciences 74, 208 (1985).
Ausgehend von 2- bzw. 3-Furylglyoxylnitril können über die daraus erhältliche 2- bzw. 3-Furylglyoxylsäure die entsprechenden Methylester auf übliche Weise hergestellt werden. Aus diesen werden wie beschrieben mit den metallorganischen Derivaten von 2-bzw. 3-Bromthiophen die entsprechenden Glykolsäureester erhalten. Die aus 2-, 3- oder 4-Halogenpyridin erhältlichen metallorganischen Verbindungen lassen sich mit 2- bzw. 3-Thienylglyoxylsäuremethylester zu den entsprechenden Glykolsäureestern umsetzen.
Thienylglykolsäureester, in denen der Thiophenring in 2- bzw. 3-Stellung Fluor enthält, werden z.B. ausgehend von 2-Fluor- bzw. 3-Fluorthiophen hergestellt (Bromieren zu 2-Brom-3-fluor- oder 2-Brom-5-fluorthiophen und, nach Überführung in entsprechende metallorganische Verbindungen, Umsetzung mit geeigneten Glyoxylsäureestern zu den Glykolsäureestern.
2-Fluorthiophen und 3-Fluorthiophen lassen sich analog Unterhalt, Arch.Pharm. 322, 839 (1989) zu den entsprechenden Glyoxylsäureestern umsetzen, die ihrerseits, wie schon beschrieben, mit z.B. 2- oder 3-Thienylderivaten, zu Glykolsäureestern umgesetzt werden können. Durch geeignete Auswahl der Komponenten lassen sich analog symmetrisch substituierte Di-thienylglykolsäureester herstellen.
Ein weiterer Weg bietet sich an analog der Benzoinkondensation und Benzilsäureumlagerung.
Die benötigten Säurechloride kann man aus den Säuren und Thionylchlorid erhalten, die Imidazolide aus den Säuren und Carbonyldiimidazol.
Die folgenden Beispiele erläutern die Erfindung, ohne sie zu beschränken.

Beispiel 1

Benzilsäurescopinester-methobromid

a) Benzilsäurescopinester aus $\alpha$-Chlordiphenylessigsäurechlorid und Scopin
In eine Lösung von 31,0 g (0,2 mol) Scopin in 60 ml wasserfreiem Pyridin werden bei 0°C innerhalb von 50 min. unter Rühren 26,5 g (0,1 mol) $\alpha$-Chlordiphenylessigsäurechlorid gegeben. Nach beendeter Zugabe wird 4 Stunden ohne Kühlung gerührt und die Mischung danach 24 Stunden stehen gelassen. Zur Aufarbeitung wird das ausgefallene Scopinhydrochlorid abgesaugt. Die abgetrennte Lösung wird unter vermindertem Druck eingedampft, der Rückstand in einem Gemisch aus 600 ml Wasser und 15 ml konz. Salzsäure gelöst und 10 min. auf ca. 80°C erhitzt. Bei einer Temperatur unter 20°C wird Natriumcarbonat zugegeben, bis pH 9 erreicht ist. Der Benzilsäurescopinester wird mit Methylenchlorid extrahiert, die Extrakte werden über Natriumsulfat getrocknet. Nach dem Einengen und Behandeln mit Aceton erhält man ein weißes Kristallisat, Fp. 182-3°C (Zers.), Ausbeute 31,8 g (87 % d.Th.).
Elementaranalyse und Spektren bestätigen das Vorliegen der Titelverbindung, die in üblicher Weise in das Hydrochlorid, Fp. 256°C (Zers.; aus Ethanol) übergeführt werden kann.

b) Benzilsäurescopinester aus Benzilsäureimidazolid und Scopin
Eine Suspension von 7,13 g (0,046 mol) Scopin und 3,2 g (0,0115 mol) Benzilsäureimidazolid in 50 ml Aceton wird zum Sieden erhitzt. Nach ca. 10 min. werden dann allmählich weitere 9,6 g (0,0345 mol) Benzilsäureimidazolid hinzugegeben. Nach beendeter Umsetzung wird mit Eis/Kochsalz abgekühlt. Die ausgefallenen Kristalle werden

abgesaugt. Es kann in das Hydrochlorid, Fp. 256°C (Zers.; aus Ethanol), übergeführt werden. Ausbeute 8,9 g, 53% d.Th.).

c) Benzilsäurescopinester-methobromid

Zu einer Suspension von 5,48 g (0,015 mol) Benzilsäurescopinester in 120 ml Acetonitril und 20 ml Tetrachlorkohlenstoff werden 7,12 g (0,075 mol) Methyl bromid, in Aceton gelöst, gegeben und unter leichtem Überdruck bis zur vollständigen Umsetzung stehen gelassen. Die ausgefallenen Kristalle werden abgesaugt, mit kaltem Acetonitril, dann mit Diethylether gewaschen und nach dem Trocknen (bei 40°C unter vermindertem Druck) aus Methanol/Ether umkristallisiert, Fp. 200°C (Zers.).
Elementaranalyse und Spektrum bestätigen das Vorliegen der gewünschten Verbindung.

Beispiel 2

1-N-β-Fluorethylnorscopolamin-methobromid

a) 1-N-β-Fluorethylnorscopolamin-hydrochlorid

Ein Gemisch aus 16,3 (0,05 mol) 1-Norscopolaminhydrochlorid, 6,3 g (0,05 mol) 2-Bromfluorethan, 10,6 g (0,1 mol) Natriumcarbonat und 100 ml Acetonitril wird 6 Stunden unter Rückfluß erhitzt. Danach werden nochmals 6,3 g (0,05 mol) 2-Bromfluorethan und 5,3 g (0,05 mol) Natriumcarbonat zugegeben und weitere 24 Stunden erhitzt. Schließlich werden nochmals 3,2 g (0,025 mol) 2-Bromfluorethan und 2,7 g (0,025 mol) Natriumcarbonat zugegeben und weitere 48 Stunden erhitzt. Man saugt ab und dampft die Lösung ein. Der Rückstand wird in Methylenchlorid aufgenommen, mit Wasser geschüttelt und die Methylenchloridphase über Natriumsulfat getrocknet. Nach dem Abdestillieren des Methylenchlorids erhält man einen öligen Rückstand, der auf übliche Weise zum Hydrochlorid umgesetzt wird. Aus Methanol/Ether erhält man 13,1 g weiße Kristalle (70,4 % d.Th.), Fp. 197-8°C (Zers.).

b) Umsetzung zum Methobromid

7,0 g (0,021 mol) des aus dem nach a) erhaltenen Hydrochlorid auf übliche Weise freigesetzten Amins werden in 20 ml abs. Acetonitril mit 9,9 g (0,104 mol) Methylbromid während 6 Tagen bei leichtem Überdruck umgesetzt. Die ausgefallenen Kristalle werden aus Methanol/Ether umkristallisiert. 3,9 g weiße Kristalle, Fp. 194°C (Zers.).
Elementaranalyse und Spektren bestätigen das Vorliegen der Titelverbindung.

Beispiel 3

Mandelsäurescopinester-methobromid

155,2 g (1,0 mol) Scopin werden in 200 ml abs. Methylenchlorid gelöst und dazu 116,9 g (0,55 mol) Acetylmandelsäurechlorid, gelöst in 100 ml abs. Methylenchlorid, getropft (bei 20°C innerhalb 1 Stunde). (Das Acetylmandelsäurechlorid erhält man aus Acetylmandelsäure und Thionylchlorid). Nach einer Stunde wird das ausgefallene Scopin-hydrochlorid abgetrennt, die Methylenchloridlösung mit Wasser extrahiert und getrocknet.

Die vereinigten Wasserphasen werden mit Natriumcarbonat alkalisch gemacht, mit Methylenchlorid extrahiert und die Methylenchloridphase getrocknet. Aus den vereinigten Methylenchloridlösungen wird das Lösungsmittel abdestilliert. Die zurückbleibende Base wird auf übliche Weise in das Hydrochlorid überführt. Nach Umkristallisieren aus Acetonitril erhält man 124,6 g (67,7 % d.Th.) weiße Kristalle, Fp. 207°C (Zers.).

27,5 g (0,075 mol) der so erhaltenen Acetylverbindung werden in 110 ml 20proz. Salzsäure 20 Stunden bei Raumtemperatur stehen gelassen. Unter Kühlen wird die Reaktionslösung alkalisch gemacht und der Mandelsäurescopinester mit Methylenchlorid extrahiert. Nach Trocknen über Natriumsulfat und Abdestillieren des Lösungsmittels wird auf übliche Weise das Hydrochlorid hergestellt.
Aus Methanol/Ether erhält man 22,5 g (92,4 % d.Th.) weiße Kristalle, Fp. 141-2°C.
10,7 g (0,037 mol) des auf übliche Weise aus dem Hydrochlorid freigesetzten Esters werden in Acetonitril mit 17,58 g (0,185 mol) Methylbromid unter leichtem Überdrück 40 Stunden stehen gelassen.

Die ausgeschiedenen Kristalle werden abgesaugt, mit kaltem Acetonitril gewaschen und aus Methanol/Ether umkristallisiert. 10,0 g (70,4 % d.Th.) weiße Kristalle, Fp. 223°C (Zers.).

Elementaranalyse und Spektren bestätigen das Vorliegen der Titelverbindung.

Beispiel 4

Xanthen-9-carbonsäure-scopinester-methobromid

a) Zu einer Lösung von 15,5 g (0,1 mol) Scopin in. 50 ml Aceton werden bei 20°C gleichzeitig eine Lösung von 11,1 g (0,11 mol) Triethylamin in 20 ml Aceton und 26,9 g (0,11 mol) Xanthen-9-carbonsäurechlorid (aus Xanthen-9-carbonsäure und Thionylchlorid) in 80 ml Aceton getropft. Nach 4 Stunden werden weitere 1,1 g (0,01 mol) Triethylamin und 2,69 (0,011 mol) Xanthen-9-carbonsäurechlorid zugegeben. Nach 4 Tagen wird abgesaugt und aus der Lösung das Lösungsmittel abdestilliert.
Zum Rückstand wird Natriumcarbonatlösung gegeben und mit Methylenchlorid extrahiert. Die organische Phase wird über Natriumsulfat getrocknet, das Lösungsmittel abdestilliert. Aus dem Rückstand erhält man in üblicher Weise das Hydrochlorid des entstandenen Xanthen-9-carbonsäurescopinesters; weiße Kristalle aus Acetonitril/Ether, Fp. 223°C (Zers.); Ausbeute 21,8 g.

b) Aus einer ausreichenden Menge des entsprechend a) erhaltenen Hydrochlorids wird in üblicher Weise die Base freigesetzt. 36,3 g (0,1 mol) davon werden in einer Lösung von 47,5 g (0,5 mol) Methylbromid in 49 g Acetonitril 24 Stunden unter leichtem Überdruck umgesetzt. Die entstandenen Kristalle werden abgesaugt, mit Aceton/Ether gewaschen und aus Ethanol umkristallisiert. Ausbeute 44,0 g (95,8 % d.Th.), weiße Kristalle, Fp. 139°C. Die Kristalle enthalten 0,5 mol Ethanol. Elementaranalyse und Spektren bestätigen das Vorliegen der Titelverbindung.

Entsprechend den vorstehenden Beispielen können auch die anderen erfindungsgemäßen Verbindungen erhalten werden. Die Verbindungen schmelzen unter Zersetzung.

## TABELLE I

Verbindungen der Formel

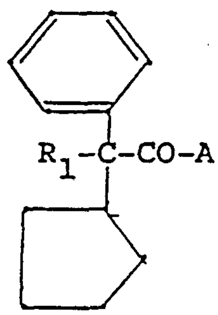

| Nr. | $R_1$ | A | Fp. [°C] |
|---|---|---|---|
| 1 | H | 3α-N-Ethyl-(6ß,7ß-Epoxy)-nortropanyl-methobromid | 228 |
| 2 | H | 3α-N-Propyl-(6ß,7ß-Epoxy)-nortropanyl-methobromid | 206-7 |
| 3 | H | 3α-N-Isopropyl-(6ß,7ß-Epoxy)-nortropanyl-methobromid | 218 |
| 4 | H | 3α-(6ß,7ß-Epoxy)-tropanyl-methobromid | 207 |
| 5 | H | 3α-(6 ,7 -Dehydro)tropanyl-methobromid | 226-8 |

## TABELLE II

Verbindungen der Formel

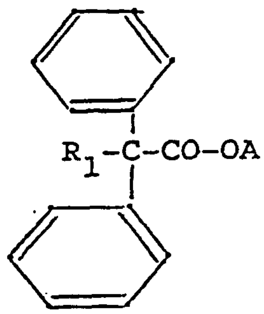

| Nr. | $R_1$ | A | Fp. [°C] |
|---|---|---|---|
| 1 | OH | 3α-(6ß,7ß-Epoxy)tropanyl-methobromid | 200 |
| 2 | OH | 3α-N-Ethyl-(6ß,7ß-Epoxy)-nortropanyl-methobromid | 220-1 |
| 3 | OH | 3α-(6ß,7ß-Epoxy)-N-isopropyl-nortropanyl-methobromid | 234-5 |
| 4 | OH | 3α-(6 ,7 -Dehydro)tropanyl-methobromid | 207-8 |
| 5 | H | 3α-(6 ,7 -Dehydro)tropanyl-methobromid | 214-5 |
| 6 | OH | 3α-(6 ,7 -Dehydro)-N-isopropyl-nortropanyl-methobromid | 223 |

## TABELLE III

Verbindungen der Formel

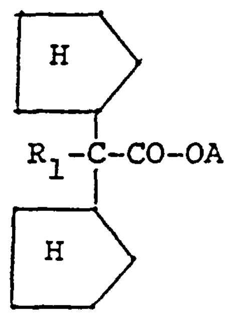

| Nr. | $R_1$ | A | Fp. [°C] |
|---|---|---|---|
| 1 | H | 3α-(6ß,7ß-Epoxy)-tropanyl-methobromid | 213-4 |
| 2 | H | 3α-(6ß,7ß-Epoxy)-tropanyl-propargochlorid | 204-5 |
| 3 | H | 3α-N-Ethyl-(6ß,7ß-Epoxy)-nortropanyl-methobromid | 201 |
| 4 | H | 3α-(6 ,7 -Dehydro)tropanyl-methobromid | 238-9 |
| 5 | OH | 3α-(6ß,7ß-Epoxy)tropanyl-metho-methansulfonat | |

## TABELLE IV

Verbindungen der Formel

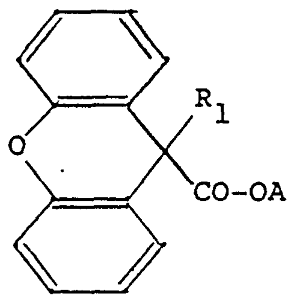

| Nr. | $R_1$ | A | Fp. [°C] |
|---|---|---|---|
| 1 | H | 3α-(6ß,7ß-Epoxy)-N-n-propyl nortropanyl-methobromid | 213-4 |
| 2 | H | 3α-(6ß,7ß-Epoxy)-N-isopropyl-nortropanyl-methobromid | 242 |
| 3 | H | 3α-(6ß,7ß-Epoxy)-N-ethyl-nortropanyl-methobromid | 217 |
| 4 | H | 3α-(6ß,7ß-Epoxy)tropanyl-methobromid (mit Kristallether) | 139 |
| 5 | H | 3α-(6ß,7ß-Epoxy)tropanyl-ethobromid | 128-31 |
| 6 | H | 3α-(6. ,7 ,-Dehydro)-N-isopropyl-nortropanyl-methobromid | 259 |
| 7 | H | 3α-(6 ,7 -Dehydro)-tropanyl-methobromid | 237-8 |

## Tabelle V

Verbindungen der Formel

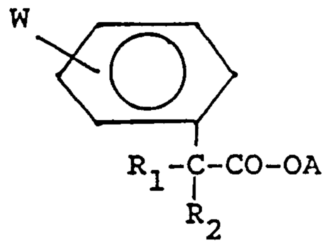

| Nr. | $R_1$ | $R_2$ | W | A | Fp[°C] |
|---|---|---|---|---|---|
| 1 | OH | H | H | 3α-(6ß,7ß-Epoxy)-tropanyl-methobromid | 223 |
| 2 | $CH_2OH$ | H | H | 3α-(6ß,7ß-Epoxy)-tropanyl-ethobromid | 190 |
| 3 | H | Cyclo-heptyl | H | 3α-(6ß,7ß-Epoxy)-tropanyl-methobromid | 212 |
| 4 | H | Cyclo-heptyl | H | 3α-(6ß,7ß-Epoxy)-tropanyl-propobromid | 215-6 |
| 5 | $-C_5H_8-$ | | H | 3α-(6ß,7ß-Epoxy)-tropanyl-methobromid | 223-4 |
| 6 | $CH_2OH$ | H | H | 3α-(6ß,7ß-Epoxy)-N-(ß-fluorethyl)-nor-tropanyl-methobromid | 194 |
| 7 | $CH_2OH$ | H | H | 3α-(6ß,7ß-Epoxy)-N-(β-hydroxyethyl)-nortropanyl-methobromid | 211 |
| 8 | OH | $C_6H_{11}$ | H | 3α-(6,7-Dehydro)-tropanyl-methobromid | 233-5 |

## Tabelle VI

Verbindungen der Formel

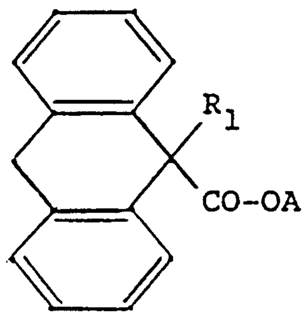

| Nr. | A | $R_1$ | Fp.[°C] |
|---|---|---|---|
| 1 | 3α-(6ß,7ß-Epoxy)-tropanyl-methobromid . $H_2O$ | H | 176 |
| 2 | 3α-(6,7-Dehydro)-tropanyl-methobromid | OH | |
| 3 | 3α-(6ß,7ß-Epoxy)-N-isopropyl-nortropanyl-methobromid | OH | |
| 4 | 3α-(6ß,7ß-Epoxy)-N-ethylnor-tropanyl-methobromid | OH | |

## Tabelle VII

Verbindungen der Formel

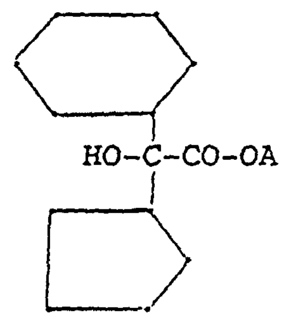

| Nr. | A | Fp.[°C] |
|---|---|---|
| 1 | 3α-(6ß,7ß-Epoxy)-tropanyl-methobromid | |
| 2 | 3α-(6,7-Dehydro)-tropanyl-methobromid | |
| 3 | 3α-(6ß,7ß-Epoxy)-tropanyl-ethobromid | |
| 4 | 3α-(6,7-Dehydro)-tropanyl-ethobromid | |

## Tabelle VIII

Verbindungen der Formel

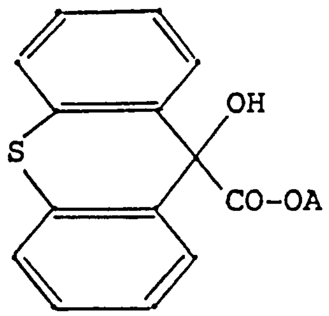

| Nr. | A | Fp. [°C] |
|---|---|---|
| 1 | 3α-(6ß,7ß-Epoxy)-tropanyl-methobromid | |
| 2 | 3α-(6,7-Dehydro)-tropanyl-methobromid | |
| 3 | 3α-(6ß,7ß-Epoxy)-tropanyl-ethobromid | |
| 4 | 3α-(6,7-Dehydro)-tropanyl-ethobromid | |

Tabelle IX

Verbindungen der Formel

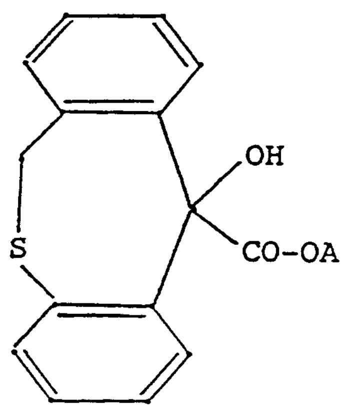

| Nr. | A | Fp.[°C] |
|---|---|---|
| 1 | 3α-(6ß,7ß-Epoxy)-tropanyl-methobromid | |
| 2 | 3α-(6,7-Dehydro)-tropanyl-methobromid | |
| 3 | 3α-(6ß,7ß-Epoxy)-tropanyl-ethobromid | |
| 4 | 3α-(6,7-Dehydro)-tropanyl-ethobromid | |

Tabelle X

Verbindungen der Formel

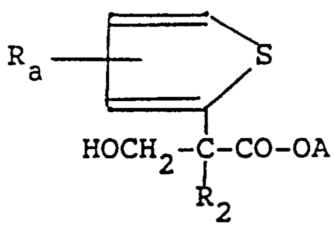

| Nr. | A | $R_2$ | $R_a$ | Fp.[°C] |
|---|---|---|---|---|
| 1 | 3α-(6ß,7ß-Epoxy)-tropanyl-methobromid | 2-Thienyl | 5-Methyl | |
| 2 | 3α-(6,7-Dehydro)-tropanyl-methobromid | 2-Thienyl | 5-Methyl | |
| 3 | 3α-(6ß,7ß-Epoxy)-tropanyl-methobromid | 2-(5-Methyl)-thienyl | 5-Methyl | |
| 4 | 3α-(6,7-Dehydro)-tropanyl-methobromid | 2-(5-Methyl)-thienyl | 5-Methyl | |
| 5 | 3α-(6ß,7ß-Epoxy)-tropanyl-methobromid | 2-Thienyl | 5-Fluor | |
| 6 | 3α-(6,7-Dehydro)-tropanyl-methobromid | 2-Thienyl | 5-Fluor | |
| 7 | 3α-(6ß,7ß-Epoxy)-tropanyl-methobromid | 2-(5-Fluor)-thienyl | 5-Fluor | |
| 8 | 3α-(6,7-Dehydro)-tropanyl-methobromid | 2-(5-Fluor)-thienyl | 5-Fluor | |

Tabelle XI

Verbindungen der Formel

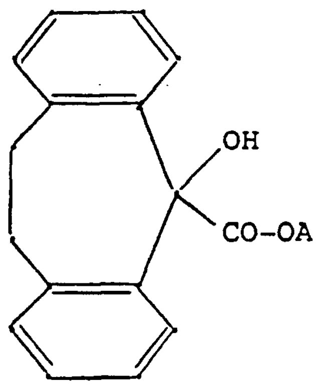

| Nr. | A | Fp.[°C] |
|---|---|---|
| 1 | 3α-(6ß,7ß-Epoxy)-tropanyl-methobromid | |
| 2 | 3α-(6,7-Dehydro)-tropanyl-methobromid | |
| 3 | 3α-(6ß,7ß-Epoxy)-N-isopropyl-nortropanyl-methobromid | |
| 4 | 3α-(6ß,7ß-Epoxy)-N-ethylnor-tropanyl-methobromid | |
| 5 | 3α-(6ß,7ß-Epoxy)-N-ß-fluor-ethyl-nortropanyl-methobromid | |
| 6 | 3α-(6ß,7ß-Epoxy)-N-n-propyl-nortropanyl-methobromid | |
| 7 | 3α(6,7-Dehydro)-tropanyl-metho-methansulfonat | |
| 8 | 3ß-(6ß,7ß-Epoxy)-tropanyl-methobromid | |
| 9 | 3ß-(6,7-Dehydro)-tropanyl-methobromid | |

## Tabelle XII

Verbindungen der Formel

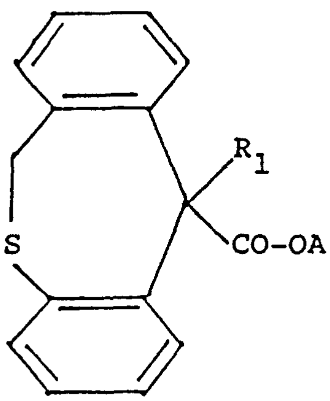

| Nr. | A | $R_1$ | Fp.[°C] |
|---|---|---|---|
| 1 | 3α-(6ß,7ß-Epoxy)-tropanyl methobromid | OH | |
| 2 | 3α-(6,7-Dehydro)-tropanyl methobromid | OH | |
| 3 | 3α-(6ß,7ß-Epoxy)-tropanyl methobromid | Methyl | |
| 4 | 3α-(6,7-Dehydro)-tropanyl methobromid | Methyl | |

## Tabelle A

Verbindungen der Formel

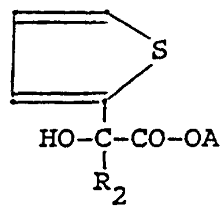

| Nr. | A | $R_2$ | Base | Fp.[°C] Hydro-chlorid |
|---|---|---|---|---|
| 1 | 3α-(6ß,7ß-Epoxy)-tropanyl | 2-Pyridyl | | |
| 2 | 3α-(6,7-Dehydro)-tropanyl | 2-Pyridyl | | |

**Patentansprüche**

**1.** Die neuen Verbindungen der Formel

A - O - CO - Z

in der

A eine Gruppe der Formel

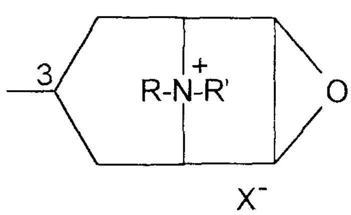

oder

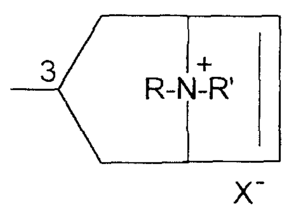

in der 3-α-Form steht, worin
einer der Reste R und R' Methyl, der andere Methyl, Ethyl, n-Propyl oder i-Propyl bedeuten

$X^-$ für ein Äquivalent eines Anions, und

Z für die Gruppe

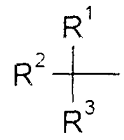

(III)

steht, worin

$R_1$ OH, Methyl oder $CH_2OH$,

$R_2$ und $R_3$, die gleich oder verschieden sein können und von denen eines auch H sein kann,

a) Phenyl-, Furyl, Thienyl, $C_5$-$C_7$-Cycloalkyl, Pyridyl, $C_5$-$C_7$-Cycloalkenyl, oder

b) einen gegebenenfalls sauerstoffunterbrochenen aliphatischen Rest mit bis zu 20 C-Atomen, einen phenyl-, phenoxy-, thienyl-, furyl-, $C_5$-$C_7$-cycloalkyl- oder fluorsubstituierten $C_1$-$C_6$-Alkylrest bedeutet,

oder die gesamte Gruppe III auch für einen tricyclischen Rest der Formel

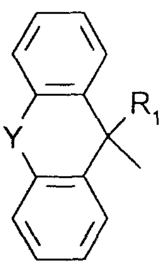

oder

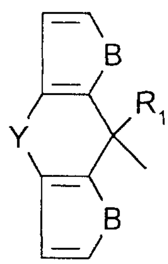

(IV) (V)

oder einen Rest der Formel

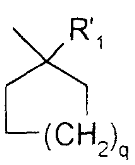

(VI)

steht, wobei B S oder CH=CH, $R'_1$ das gleiche wie $R_1$ und zusätzlich Phenyl, Thienyl, Furyl. Thiazolyl, Thiadiazolyl oder Methoxyphenyl, Y eine Einfachbindung, ein O- oder S-Atom oder eine der Gruppen $-CH_2$, $-CH_2-CH_2-$, -CH=CH-, $-OCH_2-$ oder $-SCH_2-$ und q 1, 2 oder 3 repräsentiert,
mit der Maßgabe,

a) daß Z-CO nicht für den Tropasäurerest steht, wenn A

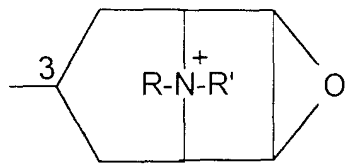

und R und R' Methyl oder R Methyl und R' Ethyl bedeuten;

b) daß wenn $R_1$ OH oder Methyl und einer der Reste $R_2$ oder $R_3$ Thienyl bedeuten, der andere der Reste $R_2$ oder $R_3$ nicht gleichzeitig Thienyl, Phenyl, Furyl, Cyclopentyl oder Cyclohexyl sein kann;

**2.** Verbindungen nach Anspruch 1 in denen die Gruppe $-CR_1R_2R_3$ (III) eine der Gruppen IV, V oder VI bedeutet.

**3.** Arzneimittel, gekennzeichnet durch einen Gehalt an einer Verbindung nach Anspruch 1 oder 2, neben üblichen Hilfs- und/oder Trägerstoffen

**4.** Verwendung von Verbindungen nach Anspruch 1 oder 2 bei der Herstellung von anticholinergen Arzneimitteln.

**5.** Verwendung von Verbindungen nach Anspruch 1 oder 2 zur Herstellung eines Arzneimittels zur Behandlung von Atemwegserkrankungen und Sinusbradykardien.

## Claims

**1.** The new compounds of formula

$$A - O - CO - Z \qquad (I)$$

wherein

A represents a group of the formula

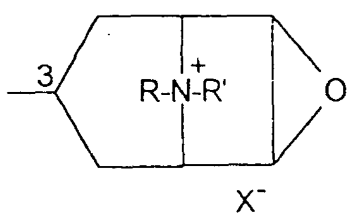

or

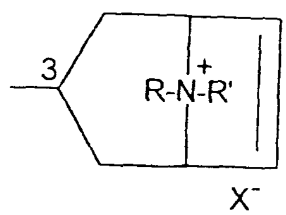

in the 3-α form, wherein
one of the groups R and R' denotes methyl, the other denotes methyl, ethyl, n-propyl or i-propyl,

$X^-$ denotes an equivalent of an anion, and

Z denotes the group

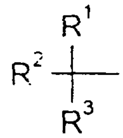

(III)

wherein

$R_1$ denotes OH, methyl or $CH_2OH$,

$R_2$ and $R_3$, which may be identical or different and one of which may also denote H,

a) phenyl, furyl, thienyl, $C_{5-7}$-cycloalkyl, pyridyl, $C_{5-7}$-cycloalkenyl, or

b) an aliphatic group having up to 20 carbon atoms optionally interrupted by oxygen, a phenyl, phenoxy, thienyl, furyl, $C_{5-7}$-cycloalkyl or fluorine-substituted $C_{1-6}$-alkyl group,

or the entire group III also represents a tricyclic group of the formula

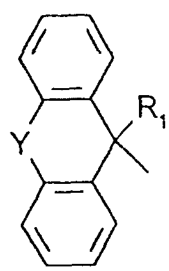

or

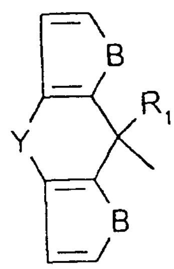

(IV) (V)

or a group of the formula

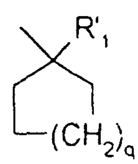

(VI)

wherein B denotes S or CH=CH, $R'_1$ means the same as $R_1$ and may additionally denote phenyl, thienyl, furyl, thiazolyl, thiadiazolyl or methoxyphenyl,

Y denotes a single bond, an O- or S-atom or one of the groups $-CH_2-$, $-CH_2-CH_2-$, CH=CH-, $-OCH_2-$ or $-SCH_2-$ and q denotes 1, 2 or 3,

with the proviso that

a) Z-CO does not denote the tropic acid group if A denotes

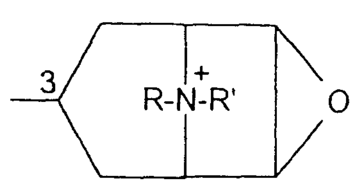

and R and R' denote methyl or R denotes methyl and R' denotes ethyl;

b) if $R_1$ denotes OH or methyl and one of the groups $R_2$ or $R_3$ denotes thienyl, the other group $R_2$ or $R_3$ cannot simultaneously be thienyl, phenyl, furyl, cyclopentyl or cyclohexyl.

**2.** Compounds according to claim 1, wherein the group $-CR_1R_2R_3$ (III) represents one of the groups IV, V or VI.

**3.** Pharmaceutical compositions, characterised in that they contain a compound according to claim 1 or 2 together with conventional excipients and/or carriers.

**4.** Use of compounds according to claim 1 or 2 in the preparation of anticholinergic pharmaceutical compositions.

**5.** Use of compounds according to claim 1 or 2 for treating respiratory tract diseases and sinus bradycardia.

**Revendications**

**1.** Nouveaux composés de formule

A - O - CO - Z

dans laquelle

A représente un groupe de formule

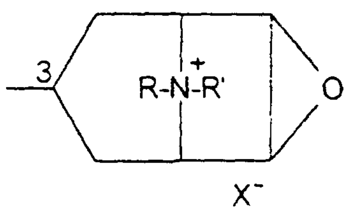

ou

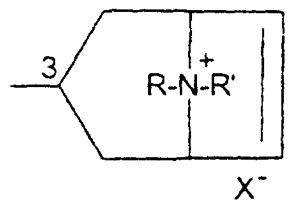

sous la forme 3-$\alpha$, où
l'un des restes R et R' représente méthyle, l'autre représente méthyle, éthyle, n-propyle ou i-propyle
$X^-$ représente un équivalent d'un anion et
Z représente le groupe

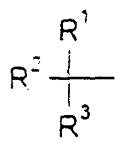

(III)

où
$R_1$ représente OH, méthyle ou $CH_2OH$,
$R_2$ et $R_3$, qui peuvent être identiques ou différents et dont l'un peut aussi être H, représentent

a) phényle, furyle, thiényle, cycloalkyle en $C_5$-$C_7$, pyridyle, cycloalcényle en $C_5$-$C_7$, ou
b) un reste aliphatique éventuellement interrompu par l'oxygène ayant jusqu'à 20 atomes de carbone, un reste phényle, phénoxy, thiényle, furyle, cycloalkyle en $C_5$-$C_7$ ou alkyle en $C_1$-$C_6$ fluorosubstitué,

ou bien le groupe III entier représente aussi un reste tricyclique de formule

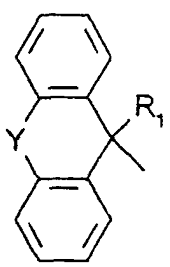

ou

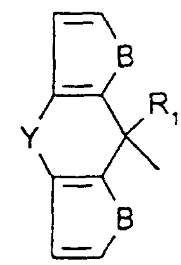

(IV) (V)

ou un reste de formule

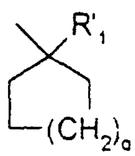

(VI)

où B représente S ou CH=CH, $R'_1$ représente la même chose que $R_1$ et en outre phényle, thiényle, furyle, thiazolyle, thiadiazolyle ou méthoxyphényle, Y représente une simple liaison, un atome O ou S ou l'un des groupes $-CH_2-$, $-CH_2-CH_2-$, -CH=CH-, $-OCH_2-$ ou $-SCH_2-$ et q représente 1, 2 ou 3
à condition

a) que Z-CO ne représente pas le reste acide tropanique quand A représente

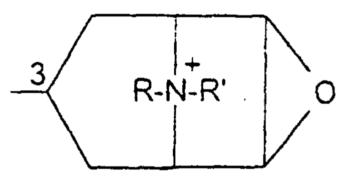

et R et R' représentent méthyle ou R représente méthyle et R' représente éthyle ;
b) que quand $R_1$ représente OH ou méthyle et l'un des restes $R_2$ et $R_3$ représente thiényle, l'autre des restes $R_2$ et $R_3$ ne puisse pas être en même temps thiényle, phényle, furyle, cyclopentyle ou cyclohexyle.

**2.** Composés selon la revendication 1 dans lesquels le groupe $-CR_1R_2R_3$ (III) représente l'un des groupes IV, V et VI.

**3.** Médicament caractérisé par une teneur en un composé selon la revendication 1 ou 2, outre des adjuvants et/ou supports courants.

**4.** Utilisation de composés selon la revendication 1 ou 2 dans la production de médicaments anticholinergiques.

**5.** Utilisation de composés selon la revendication 1 ou 2 pour la production d'un médicament pour le traitement des maladies des voies respiratoires et des bradycardies sinusales.